(19) Europäisches Patentamt
European Patent Office
Office européen des brevets

(11) Publication number: **0 188 031**
**B1**

(12) **EUROPEAN PATENT SPECIFICATION**

(45) Date of publication of patent specification: **14.09.88**

(51) Int. Cl.[4]: **C 07 C 43/225**, C 07 C 39/24, C 07 C 41/16, C 07 C 37/055

(21) Application number: **85202129.4**

(22) Date of filing: **23.12.85**

(54) **Novel substituted phenyl ethers, process for their preparation, and their use in the preparation of substituted phenols.**

(30) Priority: **09.01.85 GB 8500482**

(43) Date of publication of application:
**23.07.86 Bulletin 86/30**

(45) Publication of the grant of the patent:
**14.09.88 Bulletin 88/37**

(84) Designated Contracting States:
**AT BE CH DE FR GB IT LI LU NL SE**

(56) References cited:
**EP-A-0 004 447**
**FR-A-1 541 102**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Briner, Paul Howard**
**4 Meadowbank Cottages**
**Painters Forestal Faversham - Kent (GB)**
Inventor: **Mason, Ronald Frank**
**Kingswood - Westwell**
**Ashford - Kent (GB)**

(74) Representative: **Hunter, Keith Roger Ian et al**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 188 031 B1

Courier Press, Leamington Spa, England.

**Description**

This invention relates to novel substituted phenyl ethers, a process for their preparation, and their use in the preparation of substituted phenols.

4-Trifluoromethylphenol and 2-chloro-4-trifluoromethylphenol are useful intermediates in the preparation of certain substituted phenyl ethers — see for example US Specifications 3819755 and 3888932. These phenols are however rather difficult to synthesise, as the trifluoromethyl group tends to be unstable under a wide range of reaction conditions which might normally be used to introduce a hydroxy group into a phenyl ring. Accordingly much research has been devoted to the preparation of these compounds, and many possible routes have been described, see for example French Specification 1469596 and German Patent 1257784. All such processes, however, are either chemically or economically unattractive.

The Applicants have now found a reaction which unexpectedly gives a novel intermediate which can be used in the preparation of these substituted phenols.

The present invention provides a compound of the general formula

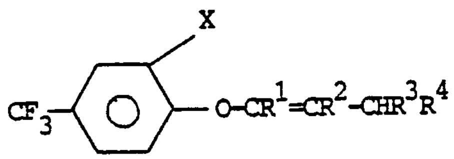

(I)

in which X represents a hydrogen or a chlorine atom, and each of $R^1$, $R^2$, $R^3$ and $R^4$ independently represents a hydrogen atom or a methyl or ethyl group.

Preferably X represents a hydrogen atom, and preferably each of $R^1$, $R^2$, $R^3$ and $R^4$ represents a hydrogen atom.

The invention also provides a process for the preparation of a compound of the general formula I, which comprises reacting a compound of the general formula

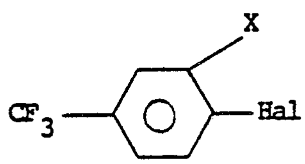

(II)

in which X has the meaning given above and Hal represents a chlorine or fluorine atom, with a compound of the general formula

$$HO—CHR^1—CR^2=CR^3R^4 \qquad (III)$$

in which $R^1$, $R^2$, $R^3$ and $R^4$ have the meanings given above, in the presence of a base.

Suitable bases include alkoxides such as sodium or potassium tertiary butoxide; alkali metal, alkaline earth metal anf quaternary ammonium hydroxides such as sodium or potassium hydroxide; and alkali metal hydrides and amides. The reaction is preferably carried out in the presence of a suitable polar aprotic solvent, for example dimethylsulphoxide, sulpholane, a polyether such as dimethoxyethane or the various oligo ethylene glycol bis ethers such as bis (2-methoxyethyl) ether (diglyme) or tetraethylene glycol dimethyl ether (tetraglyme), or an amide such as dimethyl formamide, dimethyl acetamide, hexamethylphosphoramide or N-methylpyrrolidone. Mixtures of solvents may be useful.

The reaction is preferably carried out at a temperature in the range of from 70—200°C, especially 100—150°C. The molar ratio of the reactants is not crucial; typically the molar ratio of the compound of the general formula II to the compound of the general formula III is in the range of 1:5 to 5:1, preferably 1:3 to 1:1.

It is most surprising that the process according to the present invention leads to a compound of the general formula I. It would have been expected that, on the contrary, the isomeric compound containing the group

$$—O—CHR^1—CR^2=CR^3R^4$$

would have been the product prepared by the process of the present invention. Unlike this isomeric compound, wich would be expected to be stable under acid conditions, the compound of the general formula I can be cleaved using water or an alcohol under acid conditions to produce the corresponding phenol. Accordingly the present invention also provides a process for the preparation of a compound of the general formula

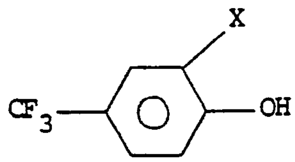

(IV)

in which X has the meaning given above, which comprises the lysis using water or an alcohol under acid conditions of a compound of the general formula I.

The compound used for the lysis may be water or a mono- or poly-functional alcohol, for example a sugar, for example galactose or glucose, or an alcohol derived from an alkane having up to 8 carbon atoms, for example ethylene glycol, glycerol, trimethylol ethane, trimethylol propane, pentaerythritol, and monoalkanols having up to 4 carbon atoms.

The water or alcohol itself may act as solvent for the reaction, or one or more additional solvents may also be used. Suitable additional solvents include for example hydrocarbons or chlorinated hydrocarbons such as carbon tetrachloride, benzene, toluene or petrol, ketones such as methyl ethyl ketone or acetone, or ethers such as dimethoxy ethane, bis(dimethoxyethane) ether or tetrahydrofuran. The acid used is not critical; mineral acids such as sulphuric, hydrochloric or phosphoric acid, organic acids such as *p*-toluene sulphonic acid, and solid ion-exchange resins, may all be used. The reaction temperature may vary over a wide range, but is preferably in the range of from 0—100°C.

If it is desired to prepare a phenol of the general formula IV in which X is a chlorine atom, this may be done by using as starting material a compound of the general formula I in which X is a chlorine atom. Alternatively, the compound may be prepared by chlorination of the phenol of the general formula IV in which X represents a hydrogen atom. This reaction may be carried out using any suitable chlorinating agent, for example N-chlorosuccinimide or elemental chlorine, preferably at a temperature in the range of from 0—150°C, conveniently under reflux or at room temperature. Any suitable solvent may be used, for example a hydrocarbon or chlorinated hydrocarbon such as those listed above.

Surprisingly, it has been found that the compound of the formula IV in which X is a chlorine atom, can be obtained easily and in high yield and high purity, if the lysis of the compound of the general formula I is carried out in the presence of an inert organic solvent, preferably a hydrocarbon or a chlorinated hydrocarbon, and in the additional presence of a polyfunctional organic alcohol containing at least two hydroxyl groups, and the resulting reaction mixture is chlorinated *in situ* using elemental chlorine. Typical alcohols which may be present are sugars, for example galactose or glucose, and alcohols derived from alkanes having up to 8 carbon atoms, for example ethylene glycol, glycerol, trimethylol ethane, trimethylol propane and, especially, pentaerythritol.

The following Examples illustrate the invention. Examples 1 to 8 illustrate the preparation of compounds of the general formula I, while Examples 9 to 16 illustrate the use of these compounds in the preparation of phenols. Example 17 illustrates the conversion of a phenol into its chlorinated analogue.

## Example 1

Allyl alcohol (174 g) was added to a mixture of potassium hydroxide (298 g of 85% flake) and sulpholane (750 ml) under nitrogen, and the mixture was heated to 125—135°C. 4-Chlorobenzotrifluoride (272 g) was added over 20 minutes at this temperature. After 2 hours, the reaction mixture was cooled to 30°C, and 207 g of propenyl 4-trifluoromethylphenyl ether was isolated by distillation from the solvent, boiling point 84—88°C at 15 mmHg.

## Example 2

A mixture of allyl alcohol (116 g) and 4-chlorobenzotrifluoride (181 g) was added over 1 hour to a mixture of dimethyl sulphoxide (500 ml) and potassium hydroxide (199 g of 85% flake) at 90°—105°C under nitrogen. The mixture was then stirred at this temperature for 3 hours after which time the temperature was reduced to 30°C, the reaction mixture was diluted with water (1l) and 60—80° petrol (750 ml). The organic phase was separated, and the solvent removed under reduced pressure. The resulting crude product weighed 204 g and was shown by GLC to contain 77% of propenyl 4-trifluoromethylphenyl ether.

## Example 3

A mixture of allyl alcohol (58 g) and 4-chlorobenzotrifluoride (91 g) was added over 2 hours to a gently refluxing (67°C) mixture of dimethylsulphoxide (250 ml), 60—80° petrol and potassium hydroxide (99.5 g of 85% flake). The mixture was then refluxed for a further 21 hours. After work-up as described above, 107.5 g of crude product, containing 91% of the desired product, was obtained. 95 g of pure propenyl 4-trifluoromethylphenyl ether, boiling point 90—95°C at 18 mm Hg, was isolated by distillation, corresponding to a yield of 86%.

## Example 4

The procedure of the preceding example was repeated except that the solvent was replaced by sulpholane (250 ml), 60—80 petrol (30 ml) and 80—100 petrol (25 ml). An 80% yield of pure propenyl 4-trifluoromethylphenyl ether was obtained.

### Example 5

A mixture of allyl alcohol (5.8 g), 4-chlorobenzotrifluoride (9.03 g) and potassium hydroxide (9.9 g of 85% flake) in 25 ml of diglyme was stirred vigorously under nitrogen for 2 hours at 120°C followed by 1 hour at 150°C. Analysis by gas chromatography showed the presence of 76% propenyl 4-trifluoromethylphenyl ether.

### Example 6

A mixture of 3,4-dichlorobenzotrifluoride (53.75 g), allyl alcohol (43.5 g) and potassium hydroxide (842.2 g of 85% pellets) in 375 ml dimethylsulphoxide was stirred at 80°C for 2 hours. The mixture was then poured into water (1100 ml) and extracted with 40/60 petrol. The main product was shown by gas chromatography to be propenyl 2-chloro-4-trifluoromethylphenyl ether.

### Example 7

A mixture of methallyl alcohol (50.48 g), 4-chlorobenzotrifluoride (63.2 g) and potassium hydroxide (69 g of 85% flake) in 175 ml sulpholane was stirred under nitrogen at 120—145°C for 21 hours. The mixture was then cooled, added to 200 ml water and extracted twice with 60/80 petroleum ether. The solvent was removed and purified by fractionation to give isobutenyl 4-trifluoromethylphenyl ether, boiling point 77—77.5°C at 8 mm Hg.

### Example 8

A mixture of allyl alcohol (4.0 kg) and 4-chlorobenzotrifluoride (6.2 kg) was added over 6 hours to a stirred mixture of potassium hydroxide (6.8 kg of 85% material) and tetraglyme (17.4 kg) at 80°C. The temperature was then raised to 100°C for 2½ hours, after which time the reaction mixture was cooled, distilled, triturated with toluene and re-distilled to give 4.7 kg of propenyl 4-trifluoromethylphenyl ether.

### Example 9

A mixture of propenyl 4-trifluoromethylphenyl ether (60.0 g), methanol (350 ml) and aqueous sulphuric acid (20 ml 98% $H_2SO_4$ in 50 ml water) was refluxed for 4 hours. After dilution with water (1200 ml), the product was extracted twice with toluene. Removal of solvent and distillation of the residue gave 4-trifluoromethylphenol (33.43 g), boiling point 63—64°C at 6 mm Hg.

### Example 10

The procedure of Example 9 was repeated except that the methanol and sulphuric acid was replaced by dimethoxyethane and hydrochloric acid. A similar result was obtained.

### Example 11

The procedure of Example 10 was repeated except that the dimethoxyethane was replaced by acetone. A similar result was obtained.

### Example 12

A mixture of propenyl 4-trifluoromethylphenyl ether (4.04 g), D-glucose (20 mmol), *p*-toluenesulphonic acid (200 mg) and carbon tetrachloride (20 ml) was refluxed with stirring under nitrogen for 2.5 hours. Analysis by gas chromatography showed complete conversion to the corresponding phenol.

### Example 13

The procedure of Example 12 was repeated except that the D-glucose was replaced by D-galactose. A similar result was obtained.

### Example 14

The procedure of Example 9 was repeated except that the propenyl ether was replaced by the corresponding isobutenyl ether. After 27 hours under reflux, gas chromatographic analysis showed that hydrolysis was 58% complete.

### Example 15

A mixture of isobutenyl 4-trifluoromethylphenyl ether (10.8 g), pentaerythritol (31 g), carbon tetrachloride (55 ml) and *p*-toluenesulphonic acid (0.5 g) was refluxed for 1 hour, after which time gas chromatographic analysis showed that conversion to the phenol was complete.

### Example 16

A mixture of propenyl 4-trifluoromethylphenyl ether (0.5 mol), pentaerythritol (0.25 mol) and *p*-toluenesulphonic acid (5 g) in carbon tetrachloride (500 ml) was stirred and refluxed under nitrogen for 40 minutes. The resulting solution was washed with water (250 ml), and then dried by azeotroping 150 ml solvent *in vacuo*. After addition of 150 ml fresh carbon tetrachloride, chlorine was passed into the solution for 2 hours, after which the solution was washed with water (250 ml) and the solvent was evaporated off. The remaining product was purified by fractional distillation to give 74.8 g of 2-chloro-4-trifluoromethylphenol, boiling point 52—53.5°C at 6 mm Hg, which was identified and shown to have a purity of greater than 97% by gas chromatography.

Example 17

4-Trifluoromethyl phenol (32.4 g) was dissolved in carbon tetrachloride (150 ml) and chlorine gas was bubbled into the stirred mixture for 1.25 hours, the temperature being maintained at 20—30°C. The solvent was then removed *in vacuo*, and 2-chloro-4-trifluoromethylphenol, boiling point 64—66°C at 10 mm Hg, was obtained by distillation, in a yield of 93%.

**Claims**

1. A compound of the general formula

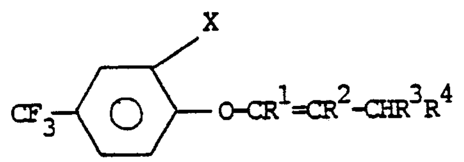

(I)

in which X represents a hydrogen or a chlorine atom, and each of $R^1$, $R^2$, $R^3$ and $R^4$ independently represents a hydrogen atom or a methyl or ethyl group.

2. A compound as claimed in claim 1, in which each of X, $R^1$, $R^2$, $R^3$ and $R^4$ represents a hydrogen atom.

3. A process for the preparation of a compound as claimed in claim 1, which comprises reacting a compound of the general formula

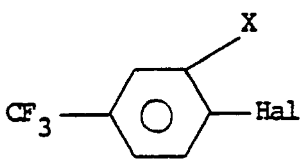

(II)

in which X has the meaning given in claim 1 and Hal represents a chlorine or fluorine atom, with a compound of the general formula

$$HO{-}CHR^1{-}CR^2{=}CR^3R^4 \quad (III)$$

in which $R^1$, $R^2$, $R^3$ and $R^4$ have the meanings given in claim 1, in the presence of a base.

4. A process as claimed in claim 3, carried out at a temperature in the range of from 100—150°C.

5. A process for the preparation of a compound of the general formula

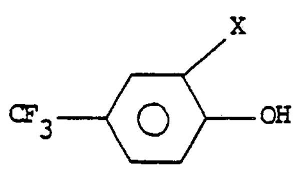

(IV)

in which X has the meaning given in claim 1, which comprises the lysis using water or an alcohol under acid conditions of a compound as claimed in claim 1.

6. A process as claimed in claim 5, in which a compound of the general formula IV is prepared in which X represents a chlorine atom, which comprises the lysis of a compound of the general formula I in which X represents a hydrogen atom, and the subsequent chlorination of the resulting compound of the general formula IV in which X represents a hydrogen atom.

7. A process as claimed in claim 6, in which the lysis of the compound of the general formula I is carried out in the presence of an inert organic solvent, and in the additional presence of a polyfunctional alcohol containing at least two hydroxyl groups, and the resulting compound of the general formula IV is chlorinated *in situ* using elemental chlorine.

**Patentansprüche**

1. Eine Verbindung der allgemeinen Formel

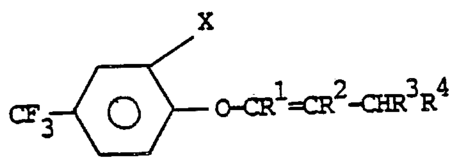

(I)

worin X ein Wasserstoff- oder ein Chloratom bedeutet und jeder der Reste $R^1$, $R^2$, $R^3$ und $R^4$ unabhägig voneinander ein Wasserstoffatom oder eine Methyl- oder Ethylgruppe darstellt.

2. Verbindung nach Anspruch 1, worin jeder der Reste X, $R^1$, $R^2$, $R^3$ und $R^4$ ein Wasserstoffatom bedeutet.

3. Verfahren zur Herstellung einer Verbindung nach Anspruch 1, welches ein Umsetzen einer Verbindung der allgemeinen Formel

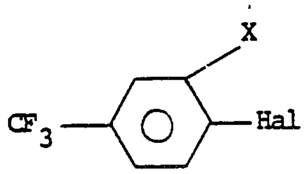

(II)

worin X die in Anspruch 1 angegebene Bedeutung aufweist und Hal ein Chlor- oder Fluoratom darstellt, mit einer Verbindung der allgemeinen Formel

$$HO—CHR^1—CR^2=CR^3R^4 \qquad (III)$$

worin $R^1$, $R^2$, $R^3$ und $R^4$ die in Anspruch 1 angegebenen Bedeutungen besitzen, in Gegenwart einer Base umfaßt.

4. Verfahren nach Anspruch 3, ausgeführt bei der Temperatur im Bereich von 100 bis 150°C.

5. Verfahren zur Herstellung einer Verbindung der allgemeinen Formel

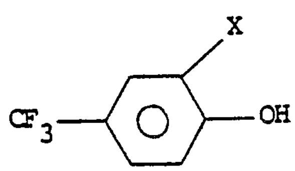

(IV)

worin X die in Anspruch 1 angegebene Bedeutung aufweist, welches eine Spaltung einer Verbindung nach Anspruch 1 unter Verwendung von Wasser oder eines Alkohols unter sauren Bedingungen umfaßt.

6. Verfahren nach Anspruch 5, worin eine Verbindung der allgemeinen Formel IV hergestellt wird, worin X ein Chloratom bedeutet, welches Verfahren die Spaltung einer Verbindung der allgemeinen Formel I, worin X ein Wasserstoffatom bedeutet, und die anschließende Chlorierung der erhaltenen Verbindung der allgemeinen Formel IV, worin X ein Wasserstoffatom bedeutet, umfaßt.

7. Verfahren nach Anspruch 6, worin die Spaltung der Verbindung der allgemeinen Formel I in Anwesenheit eines inerten organischen Lösungsmittels und in zusätzlicher Anwesenheit eines polyfunktionellen Alkohols mit einem Gehalt an wenigstens 2 Hydroxylgruppen ausgeführt wird und die erhaltene Verbindung der allgemeinen Formel IV in situ unter Verwendung von elementarem Chlor chloriert wird.

**Revendications**

1. Un composé de la formule générale

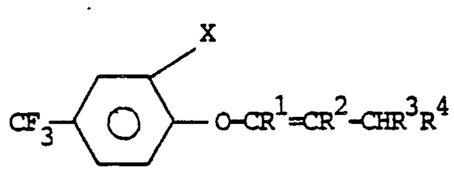

(I)

où X représente un atome d'hydrogène ou de chlore et chacun de $R^1$, $R^2$, $R^3$ et $R^4$ représente

indépendamment un atome d'hydrogène ou un groupe méthyle ou éthyle.

2. Un composé selon la revendication 1, dans lequel chacun de X, $R^1$, $R^2$, $R^3$ et $R^4$ représente un atome d'hydrogène.

3. Un procédé pour la préparation d'un composé tel que revendiqué dans la revendication 1, qui comprend la réaction d'un composé de la formule générale

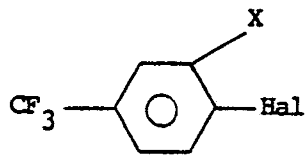

(II)

où X a la signification indiquée dans la revendication 1 et Hal représente un atome de chlore ou de fluor, avec un composé de la formule générale

$$HO—CHR^1—CR^2=CR^3R^4 \quad (III)$$

où $R^1$, $R^2$, $R^3$ et $R^4$ ont les significations indiquées dans la revendication 1, en présence d'une base.

4. Un procédé selon la revendication 3, mis en oeuvre à une température comprise entre 100 et 150°C.

5. Un procédé pour la préparation d'un composé de la formule générale

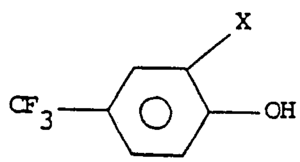

(IV)

où X a la signification indiquée dans la revendication 1, qui comprend la lyse, en utilisant de l'eau ou un alcool dans des conditions acides, d'un composé selon la revendication 1.

6. Un procédé selon la revendication 5, dans lequel on prépare un composé de la formule générale IV dans lequel X représente un atome de chlore, qui comprend la lyse d'un composé de la formule générale I dans lequel X représente un atome d'hydrogène, et la chloration ultérieure du composé résultant de la formule générale IV dans lequel X représente un atome d'hydrogène.

7. Un procédé selon la revendication 6, dans lequel la lyse du composé de la formule générale I est effectuée en présence d'un solvant organique inerte et en la présence supplémentaire d'un alcool polyfonctionnel contenant au moins deux groupes hydroxyle, et le composé résultant de la formule générale IV est chloré *in situ* en utilisant du chlore élémentaire.